# EUROPEAN PATENT SPECIFICATION

(11) **EP 1 437 944 B1**
(45) Date of publication and mention of the grant of the patent: **13.02.2008**
(21) Application number: 02778143.4
(22) Date of filing: 04.10.2002
(51) Int. Cl.: A23J 1/14, A23J 3/14, A23L 1/29

(54) **PROCESS FOR THE FRACTIONATION OF OILSEED PRESS CAKES AND MEALS**
VERFAHREN ZUR FRAKTIONIERUNG VON ÖLSAATEN-PRESSKUCHEN UND -MEHLEN
PROCEDE DE PRODUCTION DE TOURTEAUX ET D'ALIMENTS DE GRAINES OLEAGINEUSES

(30) Priority: 04.10.2001 SE 0103329
(43) Date of publication of application: 21.07.2004
(73) Proprietor: Biovelop International B.V., 1181 PJ Amstelveen (NL)
(72) Inventor: KVIST, Sten, S-260 35 Öd kra (SE); CARLSSON, Tommie, S-263 54 Höganäs (SE); LAWTHER, John, Mark, DK-4000 Roskilde (DK); BASILE DE CASTRO, Fernando, CEP-13025-061 Campinas, SP (BR)
(74) Representative: Inger, Lars Ulf Bosson
(86) International application number: PCT/SE2002/001816
(87) International publication number: WO 2003/028473

(56) References cited:
- WO-A1-98/56260
- DE-A1- 19 907 723
- GB-A- 2 127 425
- US-A- 3 402 165
- DATABASE WPI Week 199225, Derwent Publications Ltd., London, GB; Class D13, AN 1992-206820, XP002960502 & SU 1 664 245 A1 (UNIV CHERNOVTSY) 23 July 1991

## Description

### TECHNICAL FIELD

The present invention relates to a process for the fractionation of oilseed press cakes and meals.

### INTRODUCTION

The primary aim of industrial processing of oilseeds has been to maximise oil extraction. This has been achieved by using solvent extraction processes or combination of mechanical (expeller pressing) and solvent extraction. The resulting products from such extraction processes, i.e. oil and meal, have been widely used in both food and feed applications.

Even though there is no absolute agreement in the terminology used to define oilseed residues, the term oilseed meal will be used hereinafter to define the protein-fibre rich oilseed residue produced from either 1) a solvent extraction or 2) an expeller pressing and solvent extraction technology, whilst oilseed cake will be used hereinafter to define the fibre-protein-oil rich residue produced from the expeller pressing technology only.

In addition to oil and emulsifiers, oilseeds are a very interesting source of proteins, fibres and other biologically active components. In recent years, there has been a growing interest in the production and utilisation of such components. Existing commercial processes utilised for extracting such components are primarily chemical processes and targeted at oilseed meals rather than press cakes.

Large quantities of chemical-free press cakes are available in the market, which could be further processed without chemicals and yield interesting products from the technical and marketing point of view. Currently, all such press cakes are being marketed as low value commodities. Additionally, significant ton amounts of press cakes, which are currently being extracted with solvents in a second extraction step, could become available for fractionation.

This invention relates to a process for a chemical-free fractionation of oilseed meals and press cakes into at least three protein-rich fractions, at least one fibre fraction, a sugar syrup fraction, and a phytate fraction, and optionally an emulsified oil fraction.

The invention is based upon the treatment of oilseed press cakes or meals whereby the use of specific carbohydrate-degrading enzymes of the type xylanase, hemicellulase such as pentosanase, arabinase, pectinase and beta glucanase, is combined with wet milling under appropriate conditions of temperature, i.e. from 20 to 90°C, more preferably from 30 to 50°C, and pH from 4 to 6.5. The resulting hydrolysate is heated at 50 to 95°C and the above-listed fractions are separated using centrifugal separation and size-exclusion methods at such an elevated temperature. An optional fast heat treatment in a heat exchanger, specifically designed to inactivate exogenous enzymes, can be carried out either immediately after the enzymatic hydrolysis or prior to the drying of each fraction.

### PRIOR ART

Various technologies based on use of chemicals (alkali and salts) have been developed for extracting and recovering various components, in particular proteins, from oilseeds and their respective meals and cakes.

Technologies based upon the solubilisation of proteins at alkali pH followed by separation of the insoluble fraction and ultimately adjusting the pH of the protein-containing solution to their isoelectric point to cause protein precipitation has been widely demonstrated in prior art such as in patents GB 671 935; GB 900 126; EP 0 289 183; EP 0 466 524; EP 0 522 800; CN 1 121 926. Despite the acceptable protein yields obtained in such process the high chemical input, which leads to direct and indirect costs, the presence of chemical contaminants in the end-products and the loss of protein functionality due to its denaturation amongst others could be listed as the most serious handicaps of such an approach. In order to overcome or minimise protein denaturation US 4,188,399 patent discloses a process in which protein is solubilised at milder pH conditions (pH 5.1 - 5.9), the solids are separated of the liquid fraction, and the pH of the liquid fraction adjusted to match the protein isoelectric point (pH 3 - 5) for further separation of a functional protein by ultrafiltration.

No reference is made about protein yields, but the mild treatment conditions described in the said patent suggest that low yields are obtained.

US patent 1,041,717 discloses a method to solubilise and isolate vegetable proteins by using a combination of hydrogen peroxide (alkaline treatment) and enzymatic hydrolysis with proteases. The reported low protein extraction rates (<50%) and excessive use of hydrogen peroxide (up to 13.6%) have serious impact on the economy of the proposed technology.
An alternative concept to alkali solubilisation of protein followed by precipitation at the isoelectric point is that of salting out proteins. US patents 4,208,323 and 5,877,086 describe protein extraction procedures using food grade sodium chloride at specified pH and ionic strength. The above-mentioned technologies are successful in producing highly functional and non-denatured protein isolates from oil seed meals. This is, however, counterbalanced by the low protein yields and large quantities of water and salt utilised in the process.

Although most development on protein extraction of vegetable protein sources has been focused on chemical-based technologies, as described in the above-mentioned examples, other non-chemical processes have also been developed.

GB patent 598,641 informs on a process to extract proteins from leguminous protein materials previously treated with proteases. Partly hydrolysed proteins and their hydrolysis products (peptides and aminoacids) are recovered in a solution, which is further concentrated by evaporation or drying. The inventors make no reference to the use of cell wall degrading enzymes and other key inventive steps of the present invention.

A further invention on the use of enzymes to help extracting proteins from defatted solvent extracted, non-wet milled, oil seed meals is disclosed by DE 19907723 patent. The inventor refers to a method, which uses carbohydrate-degrading enzymes, prior to separation of the protein at the isoelectric point. The invention is focused exclusively on the recovery of a protein fraction (protein concentrate), which is precipitated at the isoelectric point, and a fibrous by-product. The remaining protein mass, which does not precipitate at the condition specified in the patent, remains in a sugar solution derived from the fibre hydrolysis and is regarded to be a low value by-product and as such used for animal feeding. The inventor has failed to realise that these are valuable proteins and should be recovered by other means. A further problem is that anti-nutritive factors, in particular phytates, present in the raw material will be concentrated in the by-product fraction. This will eventually lead to problems when considering animal feeding applications. No inventive steps on removal of such anti-nutritive factor have been suggested. Furthermore, the invention completely disregards the use or processing of fat-rich oilseed residues such as press cakes, and as such no proposals for fat separation have been disclosed.

A novel and alternative technology to the press expeller or solvent extraction process was developed initially for the extraction of oil from rapeseed using enzymes and wet separation (Olsen, 1987, Olsen & Christensen, 1987). The process is based on the early inactivation of the rapeseed enzymes by heat treatment. The seeds are then hydrolysed with polysaccharidases of the type pentosanase, hemicellulase, cellulase and pectinase, to disrupt the fibrous mass, thereby facilitating the subsequent removal of oil by centrifugation. In addition to oil, the process also yields protein meal, fibre (hull) and sugars as side streams, which contain various amounts of oil as a contaminant. The lower relative yield of oil compared to conventional extraction processes (10 -20% less), the low protein meal yield (<15%), and the low market price obtained from the suggested end-uses for the protein meal, hull and sugar fractions have been important obstacles for the implementation of this technology as an alternative to conventional oil extraction process. In order to reach a more attractive economy, better fractionation and more end-products with different functionalities and higher market value are needed. Additionally, removal and recovery of anti-nutritive factors such as phytate from the sugar fraction is crucial both to increase the market value of such a fraction (better nutritive value), and to yield another high value fraction (as phytate) in the process.

The inactivation or separation of anti-nutritive factors such as phytates, which can be present in considerable amounts in plant materials, is disclosed in US patent 3,736,147. The invention refers to an ultrafiltration procedure to remove phytate at various pH ranges. It is suggested the use of divalent cations, phytase enzymes or strong chelating agents at the following pH ranges, respectively, 2 - 4.5, 4.5 - 7 or 7 - 11.

It is apparent that none of the abovementioned disclosures have arrived at a chemical-free fractionation process based upon the use of polysaccharidases and designed to meet the requirements of oilseed meal and cake processing, which focus on the high recovery (up to 60%) of more than one protein fraction with different functionalities, whilst producing a fibre-rich fraction, optionally an emulsified oil fraction, sugar fraction containing significantly lower levels of phytate, and a phytate fraction.

The main objectives of this invention were to:
1. Develop a cost effective and efficient wet fractionation process to yield high value protein, fat, sugar, fibre and phytate fractions of distinct properties.
2. Combine the use of enzymatic treatment with polysaccharidase with wet milling, followed by heat treatment to facilitate the removal of solubles by centrifugal separation from the hydrolysate.
3. Maximise the recovery, after polysaccharidase treatment, of non-cell wall bound proteins into various protein-rich fraction by means of centrifugation and ultrafiltration, which exhibit rather distinct functionality and chemical composition, and therefore are suitable to different food and feed markets.
4. To maximise the extraction of a fat fraction from oilseed cakes, which consists primarily of an oil emulsion to be utilised as a high value feed additive.
5. To avoiding enzyme inactivation as a possibility so as to retrieve the enzyme cost by producing feed-grade fractions containing high polysaccharidase activity.
6. To maximise the removal of phytate originally present in the sugar fraction, without using exogenous divalent cations, phytases or chelating agents, as a means to both increase the feed value of the sugar fraction and produce another high value fraction rich in phytates.

### DESCRIPTION OF THE PREFERRED EMBODIMENTS

The inventors have developed an industrial wet fractionation process to produce, without the use of chemicals, various protein fractions, fibre, sugars with low phytate content, optionally an oil emulsion and a phytate fraction, from oilseed meals to be utilised in various market applications.

### EXAMPLE 1

Rapeseed cake obtained from press expeller process and containing 31% protein and 23.5% oil was subjected to an enzymatic hydrolysis with a multi enzyme complex containing beta glucanase, pentosanase, hemicellulase and pectinase activities in an amount of 1000 IU/g of substrate. The reaction mixture containing approximately 19% dry matter was continuously stirred and intermittently milled, at 1 hr intervals, through a wet mill to facilitate access of the enzymes into the substrate matrix and the dispersion of hydrolysis end-products. After 3 hrs of hydrolysis, the reaction mixtures was then heated up to 95°C and centrifuged whilst hot in order to separate the solubles from the precipitate fraction, which consisted primarily of hulls. The solubles were re-suspended in water and centrifuged and five layers were identified and separated, i.e. two top layers of emulsified oil, one middle layer of solubles and two bottom layers of protein fibre-rich precipitates. The soluble middle layer was then filtered through an ultrafilter fitted with 10 kDa membrane from which a retentate (protein) and a permeate (sugars) were collected. After centrifugation of the retentate a protein-rich precipitate and a supernatant were obtained. The permeate phase was centrifuged to obtain a first precipitate (phytate-rich fraction). The permeate soluble phase was then evaporated to 40° Brix and centrifuged to separate a second phyate-rich precipitate and a sugar-rich supernatant. Except the sugar supernatants, all other fractions were freeze-dried prior to analysis.

The yields of fibre, emulsified oil and sugar-rich fractions were 37.6, 16.1 and 12.8%, respectively. The protein content of the 4 protein fractions ranged from 32.6 to 92% and an overall protein extraction of 71.3% was achieved. The light phase emulsified oil was the predominant phase representing 85% of the oil phase, and consisted of 73% oil and 20% protein amongst others. The phytate content in the two phytate-rich fractions varied from 30.1 to 73%.

### EXAMPLE 2

Rapeseed cake was subjected to similar treatment conditions as described in Example 1, except that a multi enzyme complex containing twice higher hemicellulase activity was used. The extent of fibre hydrolysis was significantly higher (29%) than in Example 1. Equally, a higher protein extraction rate was achieved. The yields of fibre, emulsified oil and sugar-rich fractions were 29.5, 17.3 and 19.7%, respectively.

The results indicated that boosting fibre hydrolysis by altering specific enzyme activities improves the extraction rates of soluble components, i.e. proteins and oils.

### EXAMPLE 3

A similar trial with rapeseed cake was carried out this time with enhanced enzymatic activity against hemicelluloses and highly branched pectins. A further improvement in the fibre hydrolysis with a yield of 23% was achieved. Protein and oil overall extraction rates of 83% and 86% were achieved, and considered to be significantly superior to those described by prior art on non-chemical fractionation processes.

### EXAMPLE 4

A fractionation trial with defatted rapeseed meal (39.3% protein and 2.3% fat), which had been previously extracted by press expeller and hexane, was carried out as described in Example 1. The soluble phase from the first separation was re-suspended and centrifuged. Three layers were identified and separated, i.e. one top layer of solubles and two bottom layers of protein fibre-rich precipitates. The soluble top layer was then filtered through an ultrafilter fitted with 10KDa membrane from which a retentate (protein) and a permeate (sugars) were collected. After centrifugation of the retentate a protein-rich precipitate with 94.2% protein content and a supernatant were obtained. The permeate phase was evaporated to 40° Brix and centrifuged to separate a phytate-rich precipitate from a sugar-rich supernatant.

The yields of fibre and sugar-rich fractions were 31.3 and 17.3%, respectively. The protein content of the 4 protein fractions ranged from 33.0 to 94.2% and an overall protein extraction of 75.6% was achieved.

The recovery of the present protein fractions can contribute to an increase on the overall protein yield by as much as 100% of the expected yields in the above mentioned DE-A-19 907 723.

### END-USES

### Fibre fraction

Despite of the high extraction rate of both proteins and oil the residual insoluble fraction, hence fibre-rich fraction is still an interesting raw material for animal feeding, particularly ruminants.

Fibre fractions produced from oil seed meals and cakes contained less protein and oil than observed in the original material. The nutritive value of the fibre fraction was estimated, by full proximal analysis and "in vitro" digestibility, to be approximately 70% of raw material.

The high lignin content (15 - 27%) and the free-flowing nature of fibre fraction also indicate its potential as an energy source for biomass combustion.

### Protein fractions

Protein fractions produced by the process according to this invention have distinct composition, nutritive value and functionality. Two protein fractions extracted at early stages in the process have generally a high fibre content, which may vary from 20 to 55% depending on raw material composition and rate of hydrolysis. The protein content ranges from 30 to 65%. These protein fractions are ideal feed ingredients due to: a) high protein level and quality, b) highly digestible fibre, and c) low phytate level.

These low solubility protein fractions can also be used in food applications particularly as texturizers.

More soluble protein fractions are extracted at later stage in the fractionation process, and generally have much higher protein content, higher solubility and considerably lower fibre content. Such protein fractions may contain 45 to 95% protein, depending on process settings and raw material composition. They can also be used as feed ingredients, particularly in high value applications such as starter feed, fish feed, pet food and calf milk replacer, but should preferably be used in the functional food protein market.

### Emulsified oil fractions

The emulsified oil fractions are obtained from the fractionation of oilseed cakes. Alternatively to separating oil and the other components from this fraction, a novel end-use of the entire fraction is disclosed. The fact that it contains proteins and phospholipids, makes it an interesting source of highly digestible oil for animal feeding. This is of particular interest as an ingredient in added value compound feed containing either very high energy values or highly digestible oil.

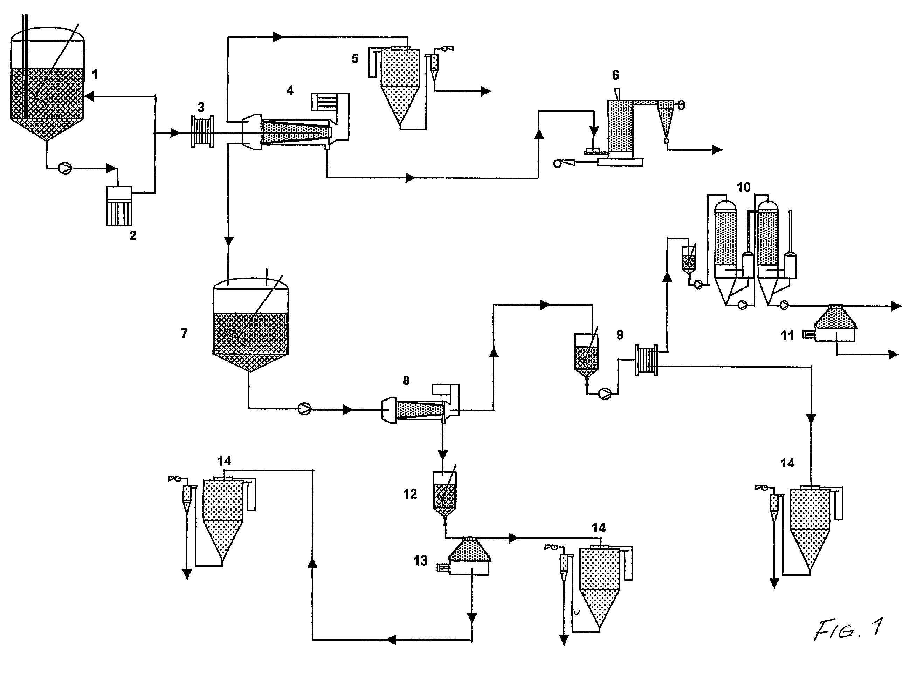

A preferred embodiment of a plant for carrying out the process of the invention is shown in the attached drawing, wherein 1 denotes a suspension, hydrolysis and heat treatment vessel 1 connected to a wet mill 2 for enhanced enzyme action and dispersion of hydrolysis end-product. The slurry is heat-treated with live steam after the completion of hydrolysis in vessel 1, and optionally further heat-treated in a heat exchanger 3 to inactivate enzymes. The enzyme inactivation step may be avoided when the end-products are targeted at the feed market. The hydrolysate with approximately 20% dry matter content is transferred to a 3-phase decanter 4, which separates fibre residue, emulsified oil and solubles. Fibre residue and emulsified oil are dried in dryers 6 and 5, respectively. A soluble phase is re-suspended in water in vessel 7 and separate in decanter 8 into two phases. The supernatant is filtered through an ultrafilter 9 to yield a permeate and a retentate phase. The permeate phase is concentrated in evaporator 10 and the resulting syrup is fractionated in separator 11 into a sugar-rich fraction and a phytate-rich fraction. The retentate phase is dried in drier 14 to yield a protein-rich fraction (60-95% protein). The precipitate from decanter 8 is re-suspended in water in vessel 12 and separated in separator 13 into two protein-rich fractions of distinct protein composition (30-65% protein), nutritive value and functionality, which are then dried in dryers 14.

## Claims

1. Process for the wet fractionation of oil seed press cake and/or meal,
**characterized in**
**that** oil seed press cake or meal is dispersed in water and subjected to a combined treatment of wet milling, enzymatic treatment by using one or a combination of more than one of the following enzymes: beta-glucanase, xylanase, hemicellulase, arabinase and pectinase at a temperature of 20-90°C, followed by a sequential fractionation at an elevated temperature of 50-95°C, using centrifugal forces and ultrafiltration so as to yield one or more fibrous-rich fractions, at least three different protein-rich fractions, optionally an oil-rich fraction, a sugar-rich fraction and a phytate-rich fraction, followed by a final step consisting of drying or partial evaporation of the above-said fractions.

2. Process according to claim 1,
wherein oil seed press cake or meal is the residual fibrous-protein fraction obtained from conventional oil extraction processes of oil seeds of the type Soya, rapeseed, cottonseed, sunflower, linseed and flax seed.

3. Process according to claims 1-2,
wherein the combination of wet milling, enzymatic and heat treatment is carried out to achieve a high efficiency in the subsequent fractionation of the main components of oilseed press-cake and meal, i.e. fibre, protein, oil, sugars and phytate, and that an extraction rate of both protein, residual fat and phytate of at least 70% from the original material is achieved.

4. Process according to claims 1-3,
wherein the hydrolysate after the wet milling is sequentially centrifuged and filtered through an ultrafilter fitted with a 10 kD membrane.

5. Process according to claim 1,
wherein an enzyme inactivation step is carried out prior to the fractionation step or drying step.

6. Process according to claims 1-6,
wherein the protein-rich fraction is provided in a dry form with at least 88% dry matter, and is comprised of one or more protein fractions produced in said process, and contains 30 to 95% protein, and 1 to 60% oil.

7. Process according to claims 1-6,
wherein the oil-rich fraction is provided as emulsified oil, and is comprised of one or two oil fractions produced in the said process, and it contains at least 60% fat, and less than 30% protein.

8. Process according to claims 1-6,
wherein the phytate-rich fraction is provided in a dry form and contains 30 to 80% phytate.

## Patentansprüche

1. Verfahren zur Nassfraktionierung von Ölsamen-Filterkuchen und/oder Essen,
**dadurch gekennzeichnet, dass**
Ölsamen-Filterkuchen oder Essen in Wasser dispergiert wird und einer kombinierten Behandlung von Nassmahlen, enzymatischer Behandlung, bei einer Temperatur von 20° - 90°C, unter Verwendung eines oder einer Kombination von mehr als einem der folgenden Enzyme unterworfen wird: Beta-glucanase, Xylanase, Hemicellulase, Arabinase und Pektinase, gefolgt von einer sequenziellen Fraktionierung bei einer erhöhten Temperatur von 50°C - 95°C unter Verwendung von zentrifugalen Kräften und Ultrafiltration, um eine oder mehrere faserreiche Fraktionen, wenigstens drei verschiedene proteinreiche Fraktionen, ggf. eine ölreiche Fraktion, eine zuckerreiche Fraktion und eine phytatreiche Fraktion zu erhalten, gefolgt von einem Schlussschritt, bestehend aus Trocknen oder teilweisem Verdampfen der vorstehend genannten Fraktionen.

2. Verfahren nach Anspruch 1, wobei Ölsamen-Filterkuchen oder Essen die verbleibende Faser-Protein-Fraktion ist, die bei herkömmlichen Ölextraktionsverfahren von Ölsamen des Typs Soja, Rapssamen, Baumwollsamen, Sonnenblumen, Leinsamen und Flachssamen erhalten wird.

3. Verfahren nach Ansprüchen 1-2, wobei die Kombination von Nassmahlen, enzymatischer und Wärme-Behandlung durchgeführt wird, um eine hohe Effizienz bei der nachfolgenden Fraktionierung der Hauptkomponenten des Ölsamen-Filterkuchens und des Essens zu erreichen, d. h. Faser, Protein, Öl, Zucker und Phytat, und damit eine Extraktionsrate von sowohl Protein, als auch verbleibendem Fett und Phytat von wenigstens 70 % des Ausgangsmaterials erreicht wird.

4. Verfahren nach Ansprüchen 1-3, wobei das Hydrolysat nach dem Nassmahlen sequentiell zentrifugiert und durch einen Ultrafilter, ausgestattet mit einer 10 kD Membran, filtriert wird.

5. Verfahren nach Anspruch 1, wobei ein Enzym-Inaktivierungsschritt vor dem Fraktionierungsschritt oder Trockenschritt durchgeführt wird.

6. Verfahren nach Ansprüchen 1-5, wobei die proteinreiche Fraktion in einer trockenen Form mit wenigstens 88 % Trockensubstanz zur Verfügung gestellt wird und in einer oder mehren Proteinfraktionen, die in dem genannten Verfahren hergestellt wurden, umfasst ist, und 30 - 95 % Protein und 1 - 60 % Öl enthält.

7. Verfahren nach Ansprüchen 1-6, wobei die ölreiche Fraktion als emulgiertes Öl zur Verfügung gestellt wird und in einer oder zwei Ölfraktionen, die in dem genannten Verfahren hergestellt werden, umfasst ist, und wenigstens 60 % Fett und weniger als 30 % Protein enthält.

8. Verfahren nach Ansprüchen 1-6, wobei die phytatreiche Fraktion in einer trockenen Form zur Verfügung gestellt wird und 30 - 80 % Phytat enthält.

## Revendications

1. Procédé pour le fractionnement humide de tourteau et/ou d'aliment de graines oléagineuses,
**caractérisé en ce que**
le tourteau ou aliment de graines oléagineuses est dispersé dans l'eau et est assujetti à un traitement combiné de mouture humide, traitement enzymatique en utilisant un enzyme ou une combinaison de plusieurs parmi les enzymes suivants : bêta - glucanase, xylanase, hémicellulase, arabinase et enzyme pectolytique selon une température de 20 à 90° C, suivi par un fractionnement séquentiel selon une température élevée de 50 à 95° C, en utilisant des forces centrifuges et l'ultrafiltration de manière à obtenir une ou plusieurs fractions riches en fibres, au moins trois fractions différentes riches en protéines, optionnellement une fraction riche en huiles, une fraction riche en sucres et une fraction riche en phytates, suivi par une étape finale consistant à faire sécher ou partiellement évaporer les fractions énoncées ci - dessus.

2. Procédé selon la revendication 1,
dans lequel le tourteau ou aliment de graines oléagineuses constitue la fraction résiduelle de fibres et protéines qui est obtenue par des procédés conventionnels d'extraction oléagineuse de graines oléagineuses du type soja, colza, semence de coton, tournesol, lin et semence de lin.

3. Procédé selon les revendications 1 et 2,
dans lequel la combinaison de mouture humide, traitement enzymatique et thermique est accomplie afin d'obtenir une grande efficacité au cours du fractionnement subséquent des composants principaux du tourteau et aliment de graines oléagineuses, c'est-à-dire des fibres, protéines, sucres et phytates, et afin d'obtenir un taux d'extraction en protéines, graisses résiduelles et phytates d'au moins 70% du matériau original.

4. Procédé selon les revendications 1 à 3,
dans lequel l'hydrolysat faisant suite à la mouture humide est séquentiellement centrifugé et filtré à travers un ultrafiltre équipé d'une membrane à perméabilité de 10 kD.

5. Procédé selon la revendication 1,
dans lequel une étape d'inactivation d'enzyme est accomplie avant l'étape de fractionnement ou l'étape de séchage.

6. Procédé selon les revendications 1 à 5,
dans lequel la fraction riche en protéines est fournie sous une forme sèche possédant au moins 88% de matière sèche et est constituée d'une ou plusieurs fractions de protéines produites au cours dudit procédé, et contient 30 à 95% de protéines et 1 à 60% d'huiles.

7. Procédé selon les revendications 1 à 7,
dans lequel la fraction riche en huiles est fournie comme une huile émulsifiée et est constituée d'une ou de deux fractions d'huile produites au cours dudit procédé, et elle contient au moins 60% de graisses et moins de 30% de protéines.

8. Procédé selon les revendications 1 à 6,
dans lequel la fraction riche en phytates est fournie sous une forme sèche et contient 30 à 80% de phytates.
